(19) Europäisches Patentamt
European Patent Office
Office européen des brevets

(11) Numéro de publication : **0 066 482**
**B1**

(12) **FASCICULE DE BREVET EUROPÉEN**

(45) Date de publication du fascicule du brevet : **28.11.84**

(51) Int. Cl.[3] : **C 07 C120/00**, C 07 C121/66, B 01 J 27/12

(21) Numéro de dépôt : **82400802.3**

(22) Date de dépôt : **03.05.82**

(54) **Procédé de préparation de nitriles aromatiques ou aliphatiques.**

(30) Priorité : **15.05.81 FR 8109694**

(43) Date de publication de la demande : **08.12.82 Bulletin 82/49**

(45) Mention de la délivrance du brevet : **28.11.84 Bulletin 84/48**

(84) Etats contractants désignés : **AT BE CH DE FR GB IT LI LU NL SE**

(56) Documents cités :
**FR-A- 1 250 165**
**Le dossier contient des informations techniques présentées postérieurement au dépôt de la demande et ne figurant pas dans le présent fascicule.**

(73) Titulaire : **RHONE-POULENC SPECIALITES CHIMIQUES**
**"Les Miroirs" 18, Avenue d'Alsace**
**F-92400 Courbevoie (FR)**

(72) Inventeur : **Jacques, Roland**
**579, Montée de Silhol**
**F-30100 Ales (FR)**
Inventeur : **Reppelin, Michel**
**10, Chemin Neuf**
**F-69660 Collonges-au-Mont-d'Or (FR)**
Inventeur : **Seigneurin, Laurent**
**3, avenue du Parc**
**F-30340 Salindres (FR)**

(74) Mandataire : **Cazes, Jean-Marie et al**
**RHONE-POULENC RECHERCHES Service Brevets Chimie et Polymères 25, quai Paul Doumer**
**F-92408 Courbevoie Cedex (FR)**

**EP 0 066 482 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention a pour objet un procédé de préparation de nitriles aromatiques ou aliphatiques.

Elle concerne plus particulièrement la préparation de nitriles de formule générale :

Ar—A—CN (I)

où Ar représente un radical benzénique et où A représente un radical hydrocarboné contenant de 1 à 6 atomes de carbone, à partir des formamides ou formanilides de formule générale :

Ar—A—NHCHO (II)

ou des amides de formule générale :

Ar—A—$CONH_2$ (III)

dans lesquelles Ar et A ont la signification précédente.

On connaît dans l'art antérieur le brevet français 1 250 165 qui décrit la préparation de nitriles à partir de composés du type (II) par réaction de ces derniers à une température comprise entre 460 et 560 °C en phase gazeuse au contact, comme catalyseurs, d'acide silicique actif ou de silicates renfermant un oxyde métallique.

Les expériences effectuées par la demanderesse avec des catalyseurs du type de ceux décrits dans le brevet français précité montrent que la sélectivité obtenue n'est pas suffisante pour une exploitation industrielle optimale du procédé. De plus, lorsque des nitriles de formule I dont le radical Ar comporte un substituant fluoré sont recherchés, on assiste à la formation de produits secondaires lourds ce qui diminue la durée de vie du catalyseur par encrassement de la surface et on observe des réactions de défluoration ce qui donne naissance à des composés très difficilement séparables du produit recherché.

Un objet de la présente invention est de fournir un procédé de préparation de nitriles aromatiques ou aliphatiques palliant les inconvénients précités de l'art antérieur.

Un autre objet de la présente invention est de fournir un procédé de préparation de nitriles aromatiques ou aliphatiques à partir d'amides de formule (III).

La présente invention a donc pour objet un procédé de préparation de nitriles aromatiques ou aliphatiques de formule :

Ar—A—CN (I)

dans laquelle Ar représente un radical benzénique et A représente un lien valentiel ou un radical hydrocarboné contenant de 1 à 6 atomes de carbone caractérisé en ce que l'on porte à une température comprise entre environ 450 °C et environ 550 °C un formamide ou formanilide de formule :

Ar—A—NHCHO (II)

ou un amide de formule :

Ar—A—$CONH_2$ (III)

où Ar et A ont la signification précédente en présence d'un catalyseur préparé par imprégnation à l'aide d'une solution aqueuse diluée d'acide fluorhydrique, ayant une concentration en HF inférieure à environ 5 % en poids, d'une silice contenant entre 0,05 et 2 % en poids de fluor exprimé en $F^-$ lié à la silice, le rapport pondéral de l'acide fluorhydrique contenu dans la solution aqueuse à la silice étant inférieur à environ 5 %, puis séchage.

On entend au sens de la présente invention par fluor lié à la silice, le fluor non combiné sous les formes connues de fluosilicates ou de fluorures.

On entend, au sens de la présente invention, par imprégnation de la silice, la mise en contact de cette dernière avec une solution aqueuse d'acide fluorhydrique.

Selon un mode de réalisation particulier, on opère par trempage de la silice dans la solution aqueuse d'acide fluorhydrique.

Selon un autre mode de réalisation particulier, on opère par pulvérisation de la solution aqueuse d'acide fluorhydrique sur la silice.

Dans l'un et l'autre cas, la quantité de solution d'acide fluorhydrique introduite dans la silice sera égale à environ le volume poreux total de la silice traitée.

La demanderesse a constaté que pour une bonne mise en œuvre de l'invention, il était préférable d'imprégner une silice ayant une surface spécifique comprise entre 200 et 300 $m^2/g$, un volume poreux

total compris entre 1 et 1,5 $cm^3/g$, un diamètre moyen des pores compris entre 100 et 200 Å ; un pH d'échange inférieur à environ 3 et une teneur en sodium exprimée en $Na_2O$ inférieure à environ 1 % en poids par rapport à la silice.

Selon un autre mode de réalisation préférentiel de l'invention, l'imprégnation est réalisée à l'aide d'une solution aqueuse d'acide fluorhydrique ayant une concentration comprise entre 0,04 % et 4 % en poids.

L'imprégnation est de préférence réalisée à température ambiante sous pression atmosphérique.

Le séchage est de préférence réalisé à une température comprise entre 150 et 600 °C pendant 1 à 24 heures environ.

La silice comprendra avantageusement après séchage au total entre 0,3 % et 3 % en poids de fluor.

Les silices à imprégner contenant de 0,05 à 2 % en poids de fluor exprimé en F peuvent être obtenues par précipitation de silicate de sodium à l'aide d'acide fluorhydrique.

Ces silices font l'objet d'une demande de brevet d'invention français au nom de la demanderesse. La précipitation du silicate de sodium à l'acide fluorhydrique se fait en ajoutant une solution aqueuse d'acide fluorhydrique à une solution aqueuse de silicate de soude, à une température comprise entre – 50 °C et 15 °C, tout en maintenant une teneur en $SiO_2$ inférieure à environ 15 % en poids du milieu réactionnel jusqu'à ce que le pH du milieu réactionnel atteigne une valeur comprise entre 3 et 4,5 ; on laisse gélifier le mélange ; on concasse en grains l'hydrogel obtenu ; on lave avec une eau dont le pH est compris entre 7 et 10 et on sèche. On utilise 1 à 1,5 mole de HF par mole de $SiO_2$. On utilise de préférence des solutions aqueuses de silicate de sodium et d'acide fluorhydrique de 40 à 50 % en poids. Le séchage se fait pendant 10 à 24 heures à 150 °C-200 °C.

Au sens de la présente invention, on entend par radical benzénique (Ar) le radical phényle et les radicaux phényle comportant un ou plusieurs substituants. On peut citer comme exemples de tels substituants les radicaux alkyle et alkoxy ayant de 1 à 6 atomes de carbone, les radicaux phényle et phénoxy et les radicaux F, $CF_3$, $OCF_3$, $SCF_3$, OH, Cl, Br, CN.

Le procédé selon l'invention est plus particulièrement adapté à la mise en œuvre de composés de formule II ou III dont le radical phényle comporte un ou plusieurs substituants fluorés F, $CF_3$, $OCF_3$ ou $SCF_3$. En effet, dans ce cas, on obtient une faible quantité de produits résultant des réactions de défluoration.

Le procédé est encore plus adapté à la mise en œuvre des composés de formule II comportant un radical fluoré. Parmi ces derniers, on peut citer le métatrifluorométhylformanilide.

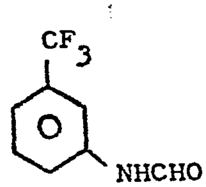

et le métatrifluorométhylbenzylformamide de formule :

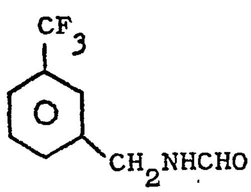

qui permettent d'obtenir respectivement le métatrifluorométhylbenzonitrile et le métatrifluorométhylphénylacétonitrile qui sont de très intéressants intermédiaires de synthèse de composés à activité phytosanitaire ou pharmaceutique.

On peut citer comme autres exemples de formule II pouvant être mis en œuvre dans le procédé de l'invention, les composés suivants : formanilide, trifluorométhyl-3 formanilide, trifluorométhyl-4 formanilide, méthoxy-4 formanilide, hydroxy-4 formanilide, fluoro-2 formanilide, fluoro-3 formanilide, fluoro-4 formanilide, chloro-2 formanilide, chloro-3 formanilide, chloro-4 formanilide, chloro-2 trifluorométhyl-5 formanilide, trifluorométhyl-3 chloro-4 formanilide, phénoxy-3 formanilide, bistrifluorométhyl-3,5 formanilide, dichloro-2,6 formanilide, difluoro-2,6 formanilide, difluoro-2,4 formanilide, trifluorométhylthio-3 formanilide, benzylformamide, trifluorométhyl-3 benzyl formamide, trifluorométhyl-4 benzylformamide, fluoro-4 benzylformamide, chloro-4 benzylformamide, fluoro-2 benzylformamide, chloro-2 benzylformamide, trifluorométhoxy-2 benzylformamide, trifluorométhoxy-4 benzylformamide, trifluorométhylthio-2 benzylformamide, trifluorométhylthio-4 benzylformamide, fluoro-2 méthyl-5 benzylformamide, fluoro-3 méthyl-6 benzylformamide, chloro-2 trifluorométhoxy-5 benzylformamide, trifluorométhoxy-2 chloro-5 benzyulformamide, difluoro-2,5 benzylformamide, difluoro-2,4 benzylformamide.

Des exemples non limitatifs de composés III sont les suivants : trifluorométhyl-3 benzamide, trifluorométhyl-4 benzamide, fluoro-2 benzamide, fluoro-3 benzamide, fluoro-4 benzamide, trifluorométhylthio-

thyl-3 phénylacétamide, fluoro-4 phénylacétamide, trifluorométhoxy-4 phényl acétamide.

On peut ainsi préparer selon le procédé de l'invention, les composés de formule I suivants : benzonitrile, trifluorométhyl-3 benzonitrile, trifluorométhyl-4 benzonitrile, méthoxy-4 benzonitrile, hydroxy-4 benzonitrile, fluoro-2 benzonitrile, fluoro-3 benzonitrile, fluoro-4 benzonitrile, chloro-2 benzonitrile, chloro-3 benzonitrile, chloro-4 benzonitrile, chloro-2 trifluorométhyl-5 benzonitrile, trifluorométhyl-3 chloro-4 benzonitrile, phénoxy-3 benzonitrile, bis-trifluorométhyl-3,5 benzonitrile, dichloro-2,6 benzonitrile, difluoro-2,6 benzonitrile, difluoro-2,4 benzonitrile, trifluorométhylthio-3 benzonitrile, trifluorométhoxy-4 benzonitrile, phénylacétonitrile, trifluorométhyl-3 phénylacétonitrile, trifluorométhyl-4 phénylacétonitrile, fluoro-4 phénylacétonitrile, chloro-4 phénylacétonitrile, fluoro-2 phénylacétonitrile, chloro-2 phénylacétonitrile, trifluorométhoxy-2 phénylacétonitrile, trifluorométhoxy-4 phényl acétonitrile, trifluorométhylthio-2 phénylacétonitrile, trifluorométhylthio-4 phénylacétonitrile, fluoro-2 méthyl-5 phénylacétonitrile, fluoro-3 méthyl-6 phénylacétonitrile, chloro-2 trifluorométhoxy-5 phénylacétonitrile, trifluorométhoxy-2, chloro-5 phénylacétonitrile, difluoro-2,5 phénylacétonitrile, difluoro-2,4 phénylacétonitrile.

Selon une manière avantageuse, mais non obligatoire, de mettre en œuvre l'invention, on opère en présence d'un diluant gazeux inerte constitué de préférence par l'azote et/ou le $CO_2$ et/ou l'acétonitrile.

On préfère utiliser l'acétonitrile en quantité telle que le pourcentage molaire de composé de formule II ou III dans l'acétonitrile soit compris entre 2 et 20 et de préférence entre 5 et 10.

La température sera, préférentiellement comprise entre 510 et 530 °C quand on mettra en œuvre un composé de formule II entre 450 et 480 °C quand on mettra en œuvre un composé de formule III.

On opère généralement à la pression atmosphérique bien que des pressions supérieures ou inférieures à la pression atmosphérique ne soient pas exclues du domaine de l'invention.

On met en œuvre le procédé selon l'invention en faisant passer, de préférence, entre 0,2 et 4 moles de composé II ou III par heure et par litre de catalyseur.

Les composés III peuvent être préparés par toute technique connue de l'homme de l'art.

La préparation des composés II quand A est un lien valentiel se fait, d'une façon bien connue dans l'art antérieur, par réaction de l'aniline correspondante avec l'acide formique.

Dans le cas où A est un radical hydrocarboné comme $—CH_2—$, les composés II peuvent être obtenus par réaction à 0-100 °C en présence d'acide fluorhydrique entre le dérivé benzénique correspondant ArH et l'hydroxyméthylformamide $OH—CH_2—NHCHO$. Le rapport de l'acide fluorhydrique à ArH est compris entre 5 et 50 et celui de ArH à $OH—CH_2—NHCHO$ est compris entre 0,5 et 2. Cette préparation fait l'objet d'une demande de brevet au nom de la demanderesse.

D'autres caractéristiques et avantages de la présente invention apparaîtront plus clairement à la lecture des exemples qui vont suivre. Ces exemples ne sauraient en aucune manière être considérés comme une limitation de l'invention.

## Exemple I

a) Préparation d'une silice contenant du fluor

On prépare une solution contenant 500 g d'HF à 40 % et 2 470 g d'eau puis une deuxième solution de silicate de soude de densité 1,185 ($SiO_2—Na_2O$ = 3,3).

Elles sont toutes les deux refroidies à 0 °C.

La solution de silicate est versée dans la solution d'acide fluorhydrique vigoureusement agitée tout en maintenant la température entre − 2 et 2 °C jusqu'à ce que le pH atteigne 4 (la concentration de la silice dans le sol est de 10,67 %).

On a alors versé 2 097 g de solution de silicate de soude. Le sol obtenu gélifie en 20 minutes. L'hydrogel est concassé de façon à obtenir des grains ayant un diamètre moyen de 2 à 6 mm. Ces grains sont plongés dans 5 fois leur volume d'eau pendant une heure, le pH de l'eau ayant été amené à 8 par introduction d'ammoniaque.

Les solides obtenus sont ensuite séchés à 200 °C pendant 24 heures. La silice est lavée par de l'eau déminéralisée puis séchée.

Les caractéristiques de la silice sont les suivantes :

| | | |
|---|---|---|
| Surface spécifique | 233 | $m^2/g$ |
| Volume poreux total | 115 | $cm^3/100$ g |
| Diamètre des pores | 115 | Å |
| Teneur en $Na_2O$ | 1 200 | ppm |
| Teneur en fluor | 0,16 % | |
| pH d'échange | 3 | |

b) Imprégnation de la silice obtenue

Les grains de silice préparés suivant a) sont laissés en contact pendant 1 heure dans de l'acide fluorhydrique à 1 % en poids puis séchés 24 h à 200 °C.

Les caractéristiques de cette silice sont les suivantes :

| | |
|---|---|
| Surface spécifique | 220 $m^2/g$ |
| Volume poreux total | 15 $cm^3/100$ g |
| Teneur en $Na_2O$ | 600 ppm |
| pH d'échange | 1,8 |
| Diamètre des pores | 120 Å |
| Teneur en F | 1,3 % |

c) Préparation de métatrifluorométhylbenzonitrile à partir de métatrifluorométhylformanilide

Dans un réacteur tubulaire en acier inxoydable d'un litre rempli de catalyseur préparé selon a et b, on introduit en continu en 450 h un mélange de 56,7 kg de métatrifluorométhylformanilide et 226,8 kg d'acétonitrile. Les débits d'alimentation sont ajustés au cours du temps de façon à maintenir un taux de conversion voisin de 95 %.

La température réactionnelle est maintenue à 520 °C tout au long du lit catalytique.

On récupère après distillation du solvant et de l'eau formée :

37,5 kg de métatrifluorométhylbenzonitrile
8,9 kg de métatrifluorométhylaniline
3,3 kg du formanilide non transformé
1,17 kg de produit lourd.

La sélectivité en nitrile est de 97,2 %.

On définit dans cet exemple et dans les exemples ultérieurs, la sélectivité comme étant le rapport du nitrile formé au formanilide ayant réagi à l'exclusion du formanilide transformé en aniline correspondante puisque cette dernière, dans un procédé industriel, peut être transformée quantitativement par l'acide formique en formanilide de départ alors recyclé.

La teneur en fluorures dans le mélange brut sortant du réacteur est de 210 ppm.

## Exemple 2

a) Imprégnation de la silice obtenue selon l'exemple 1a)

500 g de la silice préparée suivant l'exemple 1 sont introduits dans un bol tournant. On pulvérise sur les grains en rotation 550 g d'une solution aqueuse contenant 10 g d'HF. Les grains sont ensuite séchés 24 h à 200 °C.

Caractéristiques de la silice obtenue :

| | |
|---|---|
| Surface spécifique | 210 $m^2/g$ |
| Volume poreux total | 110 $cm^3/100$ g |
| Teneur en $Na_2O$ | 1 000 ppm |
| pH d'échange | 2,4 |
| Diamètre des pores | 120 Å |
| Teneur en F | 1,7 % |

b) Préparation de métatrifluorométhylphénylacétonitrile à partir de méta-trifluorométhylbenzylformamide

En opérant comme dans l'exemple 1c) on fait passer sur la silice obtenue selon 2a) à 520 °C pendant 500 heures, un mélange de 73,8 kg de métatrifluorométhylbenzylformamide et 295 kg d'acétonitrile.

On obtient après élimination de l'eau et du solvant :

56,3 kg de métatrifluorométhylphénylacétonitrile
6,60 kg de métatrifluorométhylbenzylamine
2,43 kg du formamide non transformé
1,83 kg de produits lourds.

La sélectivité en nitrile est de 97,1 %.

La teneur en fluorures dans le mélange brut est de 210 ppm.

## Exemple 3

a) Imprégnation de la silice obtenue selon l'exemple 1a)

500 g de la silice préparée suivant l'exemple 1 sont introduits dans un bol tournant. On pulvérise sur les grains en rotation 550 g d'une solution aqueuse contenant 20 g d'HF. Les grains sont ensuite séchés à 200 °C pendant 24 heures.

Caractéristiques de la silice obtenue :

| | | |
|---|---|---|
| Surface spécifique | 150 | $cm^2/g$ |
| Volume poreux total | 115 | $cm^3/100$ g |
| Teneur en $Na_2O$ | 1 200 | ppm |
| pH d'échange | 2,8 | |
| Diamètre des pores | 150 | Å |
| Teneur en F | 1,5 % | |

b) Préparation de métatrifluorométhylbenzonitrile à partir de métatrifluorométhylformanilide

En opérant comme dans l'exemple 1c), on introduit en 450 h un mélange de 42,5 kg de métatrifluorométhylformanilide et 170,1 kg d'acétonitrile. La température est maintenue à 530 °C tout au long du lit catalytique.

On récupère après distillation du solvant et de l'eau formée :

28,1 kg de métatrifluorométhylbenzonitrile
6,7 kg de métatrifluorométhylaniline
2,5 kg du formanilide non transformé
0,9 kg de produits lourds.

La sélectivité en nitrile est de 97,2 %.
La teneur en fluorures est de 250 ppm.

**Revendications**

1. Procédé de préparation de nitriles aromatiques ou aliphatiques de formule :

$$Ar—A—CN \qquad (I)$$

dans laquelle Ar représente un radical benzénique et A représente un lien valentiel ou un radical hydrocarboné contenant de 1 à 6 atomes de carbone caractérisé en ce que l'on porte à une température comprise entre 450 °C et 550 °C un formamide ou formanilide de formule :

$$Ar—A—NHCHO \qquad (II)$$

ou un amide de formule :

$$Ar—A—CONH_2 \qquad (III)$$

où Ar et A ont la signification précédente en présence d'un catalyseur préparé par imprégnation à l'aide d'une solution aqueuse diluée d'acide fluorhydrique ayant une concentration en HF inférieure à environ 5 % en poids d'une silice contenant entre 0,05 et 2 % en poids de fluor exprimé en $F^-$ lié à la silice, le rapport pondéral de l'acide fluorhydrique contenu dans la solution aqueuse à la silice étant inférieur à environ 5 %, puis séchage.

2. Procédé selon la revendication 1, caractérisé en ce qu'on effectue l'imprégnation par trempage de la silice dans la solution aqueuse d'acide fluorhydrique.

3. Procédé selon la revendication 1, caractérisé en ce qu'on effectue l'imprégnation par pulvérisation de la solution aqueuse d'acide fluorhydrique sur la silice.

4. Procédé selon l'une quelconque des revendications précédentes, caractérisé en ce que la silice contenant entre 0,05 et 2 % en poids de fluor a une surface spécifique comprise entre 200 et 300 $m^2/g$, un volume poreux total compris entre 1 et 1,5 $cm^3/g$, un diamètre moyen des pores compris entre 100 et 200 Å, un pH d'échange inférieur à environ 3 et une teneur en sodium exprimé en $Na_2O$ inférieure à environ 1 % en poids par rapport à la silice.

5. Procédé selon l'une quelconque des revendications précédentes, caractérisé en ce que l'imprégnation est réalisée à l'aide d'une solution aqueuse d'acide fluorhydrique ayant une concentration comprise entre 0,04 % et 4 % en poids.

6. Procédé selon l'une quelconque des revendications précédentes, caractérisé en ce que l'imprégnation est réalisée à température ambiante et sous pression atmosphérique.

7. Procédé selon l'une quelconque des revendications précédentes, caractérisé en ce que le séchage est effectué à une température comprise entre 150 °C et 600 °C pendant 1 à 24 h environ.

8. Procédé selon l'une quelconque des revendications précédentes, caractérisé en ce que l'on met en œuvre un composé de formule II ou III dans laquelle Ar représente un radical phényle ou un radical phényle comportant un ou plusieurs substituants choisi parmi le groupe comprenant les radicaux alkyle et alkoxy ayant de 1 à 6 atomes de carbone, phényle et phénoxy, F, $CF_3$, $OCF_3$, $SCF_3$, OH, Cl, Br, CN.

9. Procédé selon la revendication 8, caractérisé en ce que l'on met en œuvre un composé de formule II ou III dans laquelle Ar représente un radical phényle comportant un ou plusieurs substituants choisi parmi le groupe comprenant F, $CF_3$, $OCF_3$, $SCF_3$.

10. Procédé selon la revendication 9, caractérisé en ce que l'on met en œuvre le métatrifluorométhylformanilide de formule :

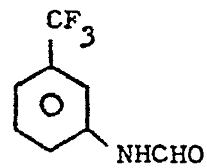

11. Procédé selon la revendication 9 caractérisé en ce que l'on met en œuvre le métatrifluorométhylbenzylformamide de formule :

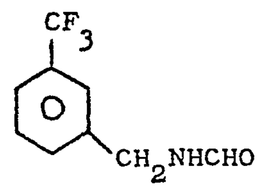

12. Procédé selon l'une quelconque des revendications précédentes caractérisé en ce que l'on fait réagir le composé de formule II ou III en présence d'un diluant gazeux inerte.

13. Procédé selon la revendication 12 caractérisé en ce que le diluant gazeux inerte est constitué par de l'azote et/ou du $CO_2$ et/ou de l'acétonitrile.

14. Procédé selon la revendication 13, caractérisé en ce que le diluant gazeux est l'acétonitrile utilisé en quantité telle que le pourcentage molaire du composé de formule II ou III dans l'acétonitrile soit compris entre 2 et 20.

**Claims**

1. Process for the preparation of aromatic or aliphatic nitriles of the formula :

$$Ar—A—CN \qquad (I)$$

in which Ar represents a benzene radical and A represents a valency bond or a hydrocarbon radical containing from 1 to 6 carbon atoms, characterised in that a formamide or formanilide of the formula :

$$Ar—A—NHCHO \qquad (II)$$

or an amide of the formula :

$$Ar—A—CONH_2 \qquad (III)$$

where Ar and A have the preceding meaning is heated to a temperature of between 450 °C and 550 °C in the presence of a catalyst, prepared by impregnating a silica, containing between 0.05 and 2 % by weight of fluorine expressed as $F^-$ bonded to the silica, with a dilute aqueous solution of hydrofluoric acid having an HF concentration less than about 5 % by weight, the weight ratio of hydrofluoric acid contained in the aqueous solution to the silica being less than about 5 %, followed by drying.

2. Process according to claim 1, characterised in that the impregnation is effected by steeping the silica in the aqueous hydrofluoric acid solution.

3. Process according to claim 1, characterised in that the impregnation is effected by spraying the aqueous hydrofluoric acid solution onto the silica.

4. Process according to any one of the preceding claims, characterised in that the silica containing between 0.05 and 2 % by weight of fluorine has a specific surface area of between 200 and 300 $m^2/g$, a total pore volume of between 1 and 1.5 $cm^3/g$, a mean diameter of the pores of between 100 and 200 Å, an exchange pH less than about 3 and a sodium content, expressed as $Na_2O$, of less than about 1 % by weight relative to the silica.

5. Process according to any one of the preceding claims, characterised in that the impregnation is effected with the aid of an aqueous hydrofluoric acid solution having a concentration of between 0.04 % and 4 % by weight.

6. Process according to any one of the preceding claims, characterised in that the impregnation is carried out at ambient temperature and under atmospheric pressure.

7. Process according to any one of the preceding claims, characterised in that the drying is effected at a temperature of between 150 °C and 600 °C for about 1 to 24 h.

8. Process according to any one of the preceding claims, characterised in that a compound of the formula II or III is employed, in which Ar represents a phenyl radical or a phenyl radical containing one or more substituents chosen from among the group comprising the alkyl and alkoxy radicals having from 1 to 6 carbon atoms and the radicals phenyl, phenoxy, F, $CF_3$, $OCF_3$, $SCF_3$, OH, Cl, Br and CN.

9. Process according to claim 8, characterised in that a compound of the formula II or III is employed in which Ar represents a phenyl radical containing one or more substituents chosen from the group comprising F, $CF_3$, $OCF_3$ and $SCF_3$.

10. Process according to claim 9, characterised in that meta-trifluoromethylformanilide of the formula :

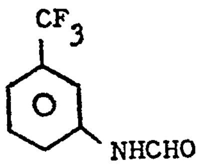

is employed.

11. Process according to claim 9, characterised in that meta-trifluoromethylbenzylformamide of the formula :

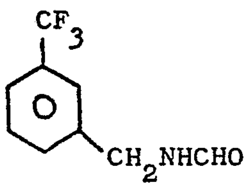

is employed.

12. Process according to any one of the preceding claims, characterised in that the compound of the formula II or III is reacted in the presence of an inert gaseous diluent.

13. Process according to claim 12, characterised in that the inert gaseous diluent consists of nitrogen and/or $CO_2$ and /or acetonitrile.

14. Process according to claim 13, characterised in that the gaseous diluent is acetonitrile used in such amount that the molar percentage of the compound of the formula II or III in the acetonitrile is between 2 and 20.

**Ansprüche**

1. Verfahren zur Herstellung von aromatischen oder aliphatischen Nitrilen der Formel :

$$Ar—A—CN \qquad (I)$$

in der Ar einen Benzolrest bedeutet und A eine Valenzbindung oder einen Kohlenwasserstoffrest mit 1 bis 6 Kohlenstoffatomen darstellt, dadurch gekennzeichnet, daß man ein Formamid oder Formanilid der Formel II :

$$Ar—A—NHCHO \qquad (II)$$

oder ein Amid der Formel III :

$$Ar—A—CONH_2 \qquad (III)$$

in denen Ar und A die vorangehende Bedeutung haben, in Gegenwart eines Katalysators, der durch Imprägnierung eines Siliciumdioxids mit einem Fluorgehalt zwischen 0,05 und 2 Gew.%, ausgedrückt als an das Siliciumdioxid gebundenes $F^-$, mit einer verdünnten wässrigen Lösung von Fluorwasserstoffsäure mit einer Konzentration an HF von weniger als etwa 5 Gew.%, wobei das Gewichtsverhältnis zwischen der

in der wässrigen Lösung enthaltenen Fluorwasserstoffsäure und dem Siliciumdioxid unter etwa 5 % liegt, und anschließende Trocknungt hergestellt wird, auf eine Temperatur zwischen 450 °C und 500 °C bringt.

2. Verfahren nach Anspruch 1, dadurch gekennzeichnet, daß man die Imprägnierung durch Tauchen des Siliciumdioxids in der wässrigen Fluorwasserstoffsäurelösung vornimmt.

3. Verfahren nach Anspruch 1, dadurch gekennzeichnet, daß man die Imprägnierung durch Versprühen der wässrigen Fluorwasserstoffsäurelösung auf das Siliciumdioxid vornimmt.

4. Verfahren nach einem der vorangehenden Ansprüche, dadurch gekennzeichnet, daß das Siliciumdioxid, das zwischen 0,05 und 2 Gew.% Fluor enthält, eine spezifische Oberfläche zwischen 200 und 300 $m^2/g$, ein Gesamtporenvolumen zwischen 1 und 1,5 $cm^3/g$, einen mittleren Porendurchmesser zwischen 100 und 200 Å, einen Austausch pH-Wert von unter ca. 3 und einen Natriumgehalt als $Na_2O$ ausgedrückt von unter etwa 1 Gew.%, bezogen auf Siliciumdioxid, besitzt.

5. Verfahren nach einem der vorangehenden Ansprüche, dadurch gekennzeichnet, daß die Imprägnierung mit Hilfe einer wässrigen Fluorwasserstoffsäurelösung vorgenommen wird, die eine Konzentration zwischen 0,04 und 4 Gew.% hat.

6. Verfahren nach einem der vorangehenden Ansprüche, dadurch gekennzeichnet, daß die Imprägnierung bei umgebungstemperaatur und unter Atmosphärendruck durchgeführt wird.

7. Verfahren nach einem der vorangehenden Ansprüche, dadurch gekennzeichnet, daß die Trocknung bei einer Temperatur zwischen 150 und 600 °C während ca. 1 bis 24 Stunden vorgenommen wird.

8. Verfahren nach einem der vorangehenden Ansprüche, dadurch gekennzeichnet, daß man eine Verbindung nach Formel II oder III einsetzt, in der Ar einen Phenylrest oder einen Phenylrest mit einem oder mehreren Substituenten aus der Gruppe der Alkyl- und Alkoxyreste mit 1 bis 6 Kohlenstoffatomen, Phenyl und Phenoxyrest, F, $CF_3$, $OCF_3$, $SCF_3$, OH, Cl, Br, CN bedeutet.

9. Verfahren nach Anspruch 8, dadurch gekennzeichnet, daß man eine Verbindung der Formel II oder III einsetzt, in der Ar einen Phenylrest mit einem oder mehreren Substituenten aus der Gruppe von F, $CF_3$, $OCF_3$, $SCF_3$ bedeutet.

10. Verfahren nach Anspruch 9, dadurch gekennzeichnet, daß man Metatrifluormethylformanilid der Formel :

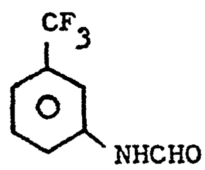

einsetzt.

11. Verfahren nach Anspruch 9, dadurch gekennzeichnet, daß man Metatrifluormethylbenzylformamid der Formel :

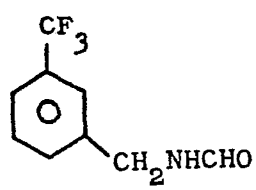

einsetzt.

12. Verfahren nach einem der vorangehenden Ansprüche, dadurch gekennzeichnet, daß man die Verbindung der Formel II oder III in Gegenwart eines inerten gasförmigen Verdünnungsmittels reagieren läßt.

13. Verfahren nach Anspruch 12, dadurch gekennzeichnet, daß das inerte gasförmige Verdünnungsmittel Stickstoff und/oder $CO_2$ und/oder Acetonitril ist.

14. Verfahren nach Anspruch 13, dadurch gekennzeichnet, daß das gasförmige Verdünnungsmittel Acetonitril ist, das in einer derartigen Menge eingesetzt wird, daß die molare Menge der Verbindung der Formel II oder III im Acetonitril zwischen 2 und 20 Prozent liegt.